# EUROPEAN PATENT APPLICATION

(11) **EP 2 522 734 A2**
(43) Date of publication of application: **14.11.2012**
(21) Application number: 12167500.3
(22) Date of filing: 10.05.2012
(51) Int. Cl.: C12N 15/89, C12M 3/00

(54) **Inoculating apparatus for biological samples in agar surface of petri capsules**

(30) Priority: 13.05.2011 ES 201130774
(71) Applicant: IUL, S.A., 08030 Barcelona (ES)
(72) Inventor: Font Castell, Victor, 08030 BARCELONA (ES)
(74) Representative: Ungria Lopez, Javier

(57) **Abstract**

The invention relates to those apparatus that include a disposable microsyringe, whose tubular body is attached by its rear end into the front starting point of a longitudinal void of an intermediate housing, locating in said longitudinal void a moving head movable upon an axis and, which is connected to the rear end of the syringe plunger. It is characterised in that the moving head (9) is also connected, by means of a longitudinal rod (12), to a moving carriage (13) movable upon an axis along the same direction as the moving head (9), and being said moving carriage (13) guided within an elongated tube (8) connected to at the rear end of the intermediate housing (7), as a continuation thereof, incorporating to said elongated tube (8) a motor element, whose actuation drives the plunger (10) by means of a moving head (9), a longitudinal rod (12) and a moving carriage (13).

## Description

### OBJECT OF THE INVENTION

The present invention, according to what has been expressed in the statement of this specification, relates to an inoculating apparatus for biological samples in the Agar surface of Petri capsules, which presents an improved characteristic structure with regard to the inoculating apparatus corresponding to the patented invention bearing the publication number ES 2126483, property of the same applicant of the invention we are dealing with.

A biological sample being submitted to an analysis to carry out a count of a micro-organisms or bacterial colonies, has to be placed in a so-called Petri capsules, according to a distribution, which can be in spiral or according to another distribution, occupying the gel or agar surface that fills the Petri capsule bottom, in which the micro-organisms have to develop.

The agar is the gelatinous environment where the microorganisms develop and it has certain texture, which should not be scratched or handled roughly by the end of the nozzle through which the inoculation of the sample is carried out. Its end has also to remain in contact with the gel to carry out a truly uniform transfer of the sample to be analyzed.

It is known that a certain weight of approximately 3 grams has not to be exceeded. The agar, due therefore to its gelatinous character, can be easily hurt if the pressure is increased, even though it is necessary to overcome the surface tension of the sample in order to damp the agar with the same.

Starting form this premise, the inoculating apparatus of the invention comprises some improvements aimed to obtain more repetitive results, avoiding that the agar is aggressed by the microsyringe and above all, achieving a longer durability of the system used to transmit motion to the microsyringe plunger.

### BACKGROUND OF THE INVENTION

At the beginning of the technique, the inoculation system of biological samples in the agar surface, suffered from a fundamental problem, which involved a significant cost increase, besides the fact that it could distort the counting of microorganism colonies.

This problem is that the inoculation (whether in spiral or not) on the gel of the Petri capsule, requires a long tube through which initially the sample travels in the aspiration direction so as to be hosted in a chamber, wherefrom later, the scheduled dosage will be poured out, by actuating a small cylinder, so the sample travels out in the opposite direction. The introduced sample to be analysed, obviously contains microorganisms and therefore after the inoculation a thorough cleaning step must be undergone to avoid contaminating the next sample.

That is why the tube that has been polluted with microorganisms, is washed with bleach, and then is rinsed out several times with water to remove any residues thereof, since if it is not very well rinsed, the bleach itself kills microorganism in subsequent analysis. This implies a severe problem that distorts the counting to a great or lesser extent.

Therefore, in this standard inoculating system a continuous tube is used, which is polluted in its entirety and that it has a cut-off or clamping valve in series, so that after being clamped, the plunger is moved to let out the desired amount of liquid.

On the other hand, the patented invention number 9600918, already mentioned in the previous section, its **characterized in that** it consist of using as inoculating device of biological samples, a syringe or microsyringe having a plunger that fills completely the chamber and which is actuated remotely, to take the sample as well as to pour it in the agar, without a significant change in the weight exerted by the syringe on the agar, and being also this syringe disposable after the inoculation, by being linked to the rest of the device in an easily removable way.

### DESCRIPTION OF THE INVENTION

With the purpose of reaching the objectives and avoiding the drawbacks mentioned in preceding sections, the invention provides an inoculating apparatus for biological samples in the agar surface of Petri capsules, of those that include a disposable microsyringe, whose tubular body is attached by its rear end into the front starting point of a longitudinal void of an intermediate housing, locating in said void a moving head moving upon an axis and which is engaged at the rear end of the plunger of the disposable microsyringe.

It is **characterized in that** the moving head is connected, by means of a longitudinal rod, to a moving carriage movable upon an axis along the same direction that the moving head, being said moving carriage guided within an elongated tube connected to the rear end of the intermediate housing, as a continuation thereof, incorporating to said elongated tube a motor element, whose actuation drives the plunger by means of a moving head, a longitudinal rod and a moving carriage.

The apparatus is also **characterized in that** the axial movement of the moving carriage is carried out by means of a motor, whose rotation outlet axle is connected to a spindle coupled in the moving carriage, being said motor located inside the rear portion of the elongated tube.

Another feature of the invention is that the tubular body of the disposable microsyringe has a rear butt having a plan shape other than circular, that is adjusted in a given position in correspondence with a complementary recess provided at the starting point of the longitudinal void of the intermediate housing, said predetermined position matching an angular outlet of the free end of the tubular body of the disposable microsyringe, being said angular outlet arranged in a plane that is perpendicular to the longitudinal direction of the disposable microsyringe, at the same time, said plane corresponds to the angle of the angular outlet making contact with the agar surface while resting completely on said surface.

The apparatus of the invention is also **characterized in that** in a particular embodiment, the rear butt of the tubular body of the disposable microsyringe presents a rectangular portion in combination with a semicircular portion.

It is also **characterized in that** the moving carriage incorporates a tab that actuates the travel length limits of said moving carriage.

Another characteristic of the invention is that the elongated tube has a longitudinal grooving through which the tab sticks out.

The apparatus is also **characterized in that** it includes a radial support, which supports the intermediate housing and elongated tube assembly, as well as the rest of the elements associated to said intermediate housing and elongated tube. The radial support has at least a first rest stable position where the disposable microsyringe is arranged with its angular outlet above the agar surface and a second working position with the angular outlet of the disposable microsyringe resting on the agar surface with a slight controlled pressure.

The radial support is **characterized in that** it comprises an arm that has at an end section thereof, a transversal opening where a portion of the elongated tube is guided and fitted, incorporating to said end section stable attaching means of the elongated tube with regard to the radial support, having also such radial support a transversal tilting axle which has at its extremities some end bearing seats.

Another characteristic of the invention is that the radial support incorporates an end counterweight guided on a bar, which has its point of origin at the arm and transversal tilting axle confluence.

The axial positioning of the end counterweight is fixed by means of the pressure exerted by a transversal screw, which is screwed into a side hole provided in the end counterweight, by pressing said transversal screw against the bar.

The stable attaching means of the elongated tube with regard to the radial support consist of an upper screw coupled into a threaded hole provided in the end section of the radial support arm.

The portion of the elongated tube whereby it is linked to the radial support, constitutes the centre of gravity of the assembly of elements, which is made up by said elongated tube, an intermediate housing, a disposable microsyringe and the remaining internal pieces associated to such elements.

Therefore, to adjust the tip pressure (angular outlet) of the disposable microsyringe on the agar, the centre of gravity of the intermediate housing and elongated tube assembly, as well as the remaining elements associated thereto, said centre of gravity is linked into the respective end section of the arm which ends by its opposite end in the transversal tilting axle, around which the apparatus of the invention can rotate supported by the two end seat bearings, counterbalancing the intended balance, if necessary, with said end counterweight, which is adjustable and is associated to the radial support arm, as it has been mentioned above.

Next, for a better comprehension of this specification and making integral part thereof, a set of figures is attached wherein, by way of illustration only and not by way of limitation, the object of the invention has been represented.

### BRIEF DESCRIPTION OF THE DRAWINGS

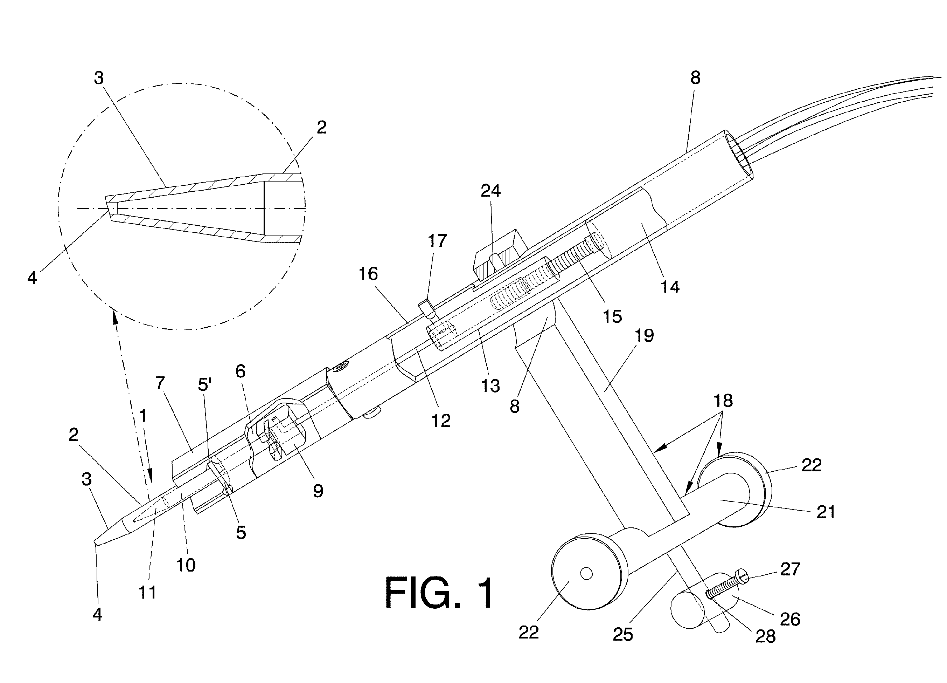

**Figure 1**.- it shows a perspective view of the inoculating apparatus for biological samples in the agar surface of Petri capsules, object of the invention.

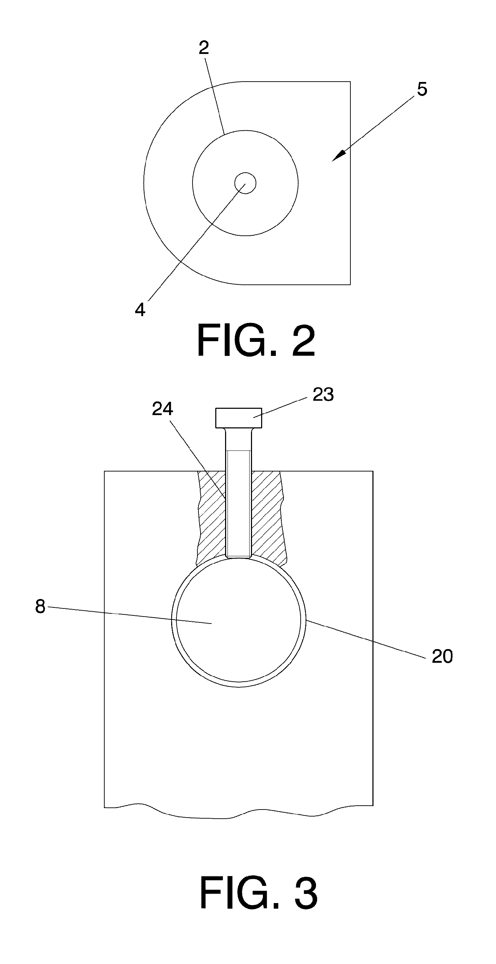

**Figure** 2.- it shows a front view of the apparatus of the invention.
**Figure 3****.**- it shows another front view of the apparatus of the invention.

### DESCRIPTION OF A PREFERRED EMBODIMENT

Taking into account the numerals adopted in the figures, the inoculating apparatus for biological samples in the agar surface of Petri capsules, provides the following nomenclature used in the description:
1.- Disposable microsyringe,
2.- Tubular body,
3.- Conical nozzle,
4.- Angular outlet,
5.- Rear butt,
5'.- Recess,
6.- Longitudinal void,
7.- Intermediate housing,
8.- Elongated tube,
9.- Moving head,
10.- Plunger,
11.- Conical end,
12.- Longitudinal rod,
13.- Moving carriage,
14.- Motor,
15.- Spindle,
16.- Longitudinal grooving,
17.- Tab,
18.- Radial support,
19.- Arm,
20.- Transversal opening,
21.- Transversal tilting axle,
22.- End seat bearings,
23.- Upper screw,
24.- Threaded hole,
25.- Bar,
26.- End counterweight,
27.- Transversal screw,
28.- Side hole,

It comprises a microsyringe 1 defined by a narrow tubular body 2, whose front end finishes in a conical nozzle 3 having an angular outlet 4 arranged in a plane that is not perpendicular to the longitudinal direction of the microsyringe 1, whilst the rear end of the narrow tubular body 2 of the disposable microsyringe 1 finishes in a rear butt 5 fixed into a recess 5' complementary of the initial section of a longitudinal void 6 of an intermediate housing 7, which is in turn bonded by its final end to an elongated tube 8 arranged along the same direction as the microsyringe 1 and the intermediate housing 7 in whose longitudinal void 6 is located and guided a moving head 9, in which the plunger 10 of the disposable microsyringe 1 is engaged, in such a way that in the plunger 10 rest position, it occupies the entire inner space of the tubular body 2 where such plunger 10 is located and guided, which also has a conical end 11 in accordance with the conical nozzle 3 of the tubular body 2 of the disposable microsyringe 1.

The rear butt 5 of the tubular body 2 presents a configuration other than a circular shape, in accordance with the recess 5', where it is located and, also in accordance with the cross-section of the longitudinal void 6 of the intermediate housing 7, achieving thus a fixed working position, defined in accordance with the angular outlet 4 that makes contact with the agar surface, which will allow it to rest completely on the agar surface and to spread perfectly the sample over said agar surface.

Particularly, the rear butt 5 of the tubular body 2 presents a rectangular portion combined with a semicircular portion.

The plunger 10 engages through its rear end into the moving head 9, which in turn connects the latter, by means of a longitudinal rod 12, to a moving carriage 13 axially guided within the elongated tube 8, in whose end section is fastened a motor 14 whose outlet axle connects to a spindle 15 that is screwed to the moving carriage 13, in such a way that when the motor 14 rotates, the rotation of the spindle 15 drives the moving carriage 13 axially, driving this one the plunger 10 in both directions depending on the direction of rotation of said motor 14, by means of a longitudinal rod 12 and a moving head 9.

The motor 14 incorporates a pulse decoder to control its rotation and the number of revolutions in accordance with the travel length of the plunger 10.

The elongated tube 8 incorporates a longitudinal grooving 16, through which sticks out a tab 17, which is bonded to the moving carriage 13. The tab 17 actuates the drive length limits of said moving carriage 13 and, therefore the plunger 10 of the disposable syringe 1.

The assembly of the apparatus of the invention is completed with a radial support 18 which supports all the described elements, tilting from a stable rest position (with the tip or angular outlet 4 end of the disposable microsyringe above the agar surface) to a stable working position (with the tip of the disposable microsyringe 1 resting on the agar surface).

The radial support 18 comprises an arm 19, whose one end section thereof incorporates a transversal opening 20 where a portion of the elongated tube 8 is adjusted. Said portion corresponds to the center of gravity of the assembly made up by an intermediate housing 7, an elongated tube 8 and the remaining elements associated thereto, while the opposing section of said arm 19 finishes in a transversal tilting axle 21 with two end seat bearings 22, which also make part of the radial support 18, as well as the transversal tilting axle 21.

The stable attachment of the elongated tube 8 with regard to the radial support 18 is carried out by means of an upper screw 23 whose screwing ensures the immobility between the radial support 18 and the elongated tube 8, whereas when said upper screw 23 is unscrewed said radial support recovers its mobility with regard to the elongated tube 8.

The radial support 18 has a bar 25, which has its point of origin at the confluence of the transversal tilting axles 21 and 19, being coupled into said bar 25 through a guide, an end counterweight 26, fixing its stable position by means of a transversal screw 27 that is screwed into a side hole 28 of said counterweight 26.

With this described arrangement, a slight tilting of the assembly apparatus of the invention around a transversal tilting axle 21 supported by the end seat bearings 22 is achieved, without this affecting the agar surface in a sample seeding position. All this is possible by regulating the end counterweight 26 and linking the arm 19 to the portion of the elongated tube 8, portion that corresponds to the center of gravity of the assembly made up by said elongated tube 8, an intermediate housing 7 and the remaining pieces associated to said elements.

Therefore, a slight tilting that does not affect the agar surface in a sample seeding position is thus achieved. The transversal tilting axle 21 can be provided with an end counterweight 26, in such a way that it is optional and is intended to fix the maximum pressure that the agar surface can stand without breaking when the angular outlet 4 of the disposable microsyringe rests on said surface.

The stable attachment of the elongated tube 8 with regard to the radial support 18 is carried out by means of an upper screw 23 coupled into a threaded hole 24 provided at the end section of the arm 19 of the radial support 18.

## Claims

1. Inoculating apparatus for biological samples in agar surface of petri capsules, being the apparatus of those that include a disposable microsyringe, whose tubular body is attached by its rear end into the front starting point of a longitudinal void of an intermediate housing, locating in said longitudinal void a moving head movable upon an axis and, which is connected to the rear end of the disposable microsyringe plunger, **characterised in that** the moving head (9) is connected, by means of a longitudinal rod (12), to a moving carriage (13) movable upon an axis along the same direction than the moving head (9), being said moving carriage (13) guided within an elongated tube (8) connected at the rear end of the intermediate housing (7), as a continuation thereof, whilst the moving head (9) is guided within the intermediate housing (7), incorporating to said elongated tube (8) a motor element, whose actuation drives the plunger (10) by means of the moving head (9), the longitudinal rod (12) and the moving carriage (13).

2. Inoculating apparatus for biological samples in agar surface of petri capsules, according to claim 1, **characterised in that** the axial movement of the moving carriage (13) is carried out by means of a motor (14) whose rotation outlet axle connects with a spindle (15) coupled into the moving carriage (13), being said motor (14) located inside the rear part of the elongated tube (8).

3. Inoculating apparatus for biological samples in agar surface of petri capsules, according to any one of the preceding claims, **characterised in that** the tubular body (2) of the microsyringe (1) has a rear butt (5) with a plan shape other than circular that is adjusted in a predetermined position in correspondence with a complementary recess (5') provided at the starting point of the longitudinal void (6) of the intermediate housing (7), corresponding said predetermined position with an angular outlet (4) of the free end of the tubular body (2) of the disposable microsyringe (1), being said angular outlet (4) arranged in a plane that is not perpendicular to the longitudinal direction of the disposable microsyringe (1) and, at the same time, said plane corresponds to the angle of the angular outlet (4) making contact with the agar surface while resting completely on said surface.

4. Inoculating apparatus for biological samples in agar surface of petri capsules, according to claim 3, **characterised in that** the rear butt (5) of the tubular body (2) of the disposable microsyringe (1) presents a rectangular portion in combination with a semicircular portion.

5. Inoculating apparatus for biological samples in agar surface of petri capsules, according to any one of the preceding claims, **characterised in that** the moving carriage (13) incorporates a tab (17) which actuates the drive length limits of said moving carriage (13).

6. Inoculating apparatus for biological samples in agar surface of petri capsules, according to claim 5, **characterised in that** the elongated tube 8 has a longitudinal grooving (16) through which the tab (17) sticks out.

7. Inoculating apparatus for biological samples in agar surface of petri capsules, according to claim 1, **characterised in that** it includes a radial support (18) made up by an arm (19) having at an end section thereof a transversal opening (20) wherein a portion of the elongated tube (8) is guided and fitted, incorporating in said end section stable attachment means of the elongated tube (8) with regard to the radial support (18), having also said radial support (18) a transversal tilting axle (21) that has at its extremities end seat bearings (22).

8. Inoculating apparatus for biological samples in agar surface of petri capsules, according to claim 7, **characterised in that** the radial support (18) incorporates an end counterweight (26) guided in a bar (25), which has its point of origin at the confluence of the arm (19) and the transversal tilting axle (21).

9. Inoculating apparatus for biological samples in agar surface of petri capsules, according to claim 8, **characterised in that** the axial positioning of the end counterweight (26) is fixed by means of the pressure exerted by a transversal screw (27) that is screwed into a side hole (28) provided in the end counterweight (26), by pressing said transversal screw (27) against the bar (25).

10. Inoculating apparatus for biological samples in agar surface of petri capsules, according to claim 7, **characterised in that** the stable attachment means of the elongated tube (8) with regard to the radial support (18), consist of an un upper screw (23) coupled in a threaded hole (24) provided at the end section of the arm (19) of the radial support (18).

11. Inoculating apparatus for biological samples in agar surface of petri capsules, according to claim 7, **characterised in that** the portion of the elongated tube (8) whereby is linked to the radial support (18), constitutes the centre of gravity of the assembly of elements made up by said elongated tube (8), the intermediate housing (7), a disposable microsyringe (1) and the remaining internal pieces associated to said elements.
